(19) Europäisches Patentamt
European Patent Office
Office européen des brevets

(11) Veröffentlichungsnummer : **0 095 638**
**B1**

(12) # EUROPÄISCHE PATENTSCHRIFT

(45) Veröffentlichungstag der Patentschrift : **28.08.85**

(21) Anmeldenummer : **83104789.9**

(22) Anmeldetag : **16.05.83**

(51) Int. Cl.[4] : **C 07 C121/78**, C 07 C149/42, C 07 D275/04, C 09 B 29/045// C07C153/063, C07C147/12

(54) **2,6-Dicyananiline.**

(30) Priorität : **28.05.82 DE 3220117**

(43) Veröffentlichungstag der Anmeldung : **07.12.83 Patentblatt 83/49**

(45) Bekanntmachung des Hinweises auf die Patenterteilung : **28.08.85 Patentblatt 85/35**

(84) Benannte Vertragsstaaten : **CH DE FR GB LI**

(56) Entgegenhaltungen :
**DE-A- 1 543 620**
**DE-A- 2 617 807**
**Chemical Abstracts Band 95, Nr. 9, 31. August 1981, Columbus, Ohio, USA K. GEWALD et al. "Syntheses of 3-amino-2,4-dicyanothiophenols using derivatives of 2-amino-3-cyanothiopyran-6-ylidencyanoacetic acid", Seite 753, Spalte 1, Abstract Nr. 80621c**
**Chemical Abstracts Band 83, Nr. 13, 29. September 1975, Columbus, Ohio, USA B. RIGERTE et al. "Properties of 2,6-dicyano-3,5-dimethylaniline", Seite 508, Spalte 2, Abstract Nr. 113891y**

(73) Patentinhaber : **BAYER AG**
**Konzernverwaltung RP Patentabteilung**
**D-5090 Leverkusen 1 Bayerwerk (DE)**

(72) Erfinder : **Hamprecht, Rainer, Dr.**
**Im Kerberich 25**
**D-5068 Odenthal (DE)**

**EP 0 095 638 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bereits bekannt, daß 2,6-Dicyananiline Ausgangsstoffe zur Herstellung von Azofarbstoffen sind (vgl. DE-A 15 43 620).

Es wurde nun gefunden, daß sich Anilinderivate der Formel

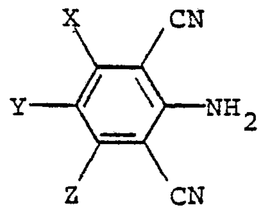

(I)

worin

X Alkyl,

Y —S-Alkyl, —$SO_2$-Alkyl, —$CO_2$—R oder — falls Z = H— auch Brom

Z Wasserstoff oder Alkyl bedeuten,

wobei

R für Alkyl, Cycloalkyl oder Wasserstoff steht und die Alkylreste 1-6, vorzugsweise 1-4 C-Atome und die Cycloalkylreste 5 oder 6 Ring-C-Atome aufweisen,

ebenfalls hervorragend zur Herstellung von wertvollen Azofarbstoffen durch Diazotierung und Kupplung eignen.

Besonders bevorzugte Alkylreste sind Methyl und — im Falle des Carbonsäureesters — auch Ethyl. Bevorzugter Cycloalkylrest ist Cyclohexyl.

Bevorzugte Anilinderivate sind solche der Formel I, worin

X Methyl und

Z Wasserstoff bedeuten.

Ganz besonders bevorzugt sind Verbindungen der Formel I, worin

X und Z die vorstehend genannten allgemeinen und besonderen Bedeutungen haben und

Y für —$CO_2$-$C_1$-$C_6$-Alkyl steht.

Während 2,6-Dicyananiline mit einem Nitro-, Brom- oder Alkyl-Substituenten in 4-Stellung, einem Alkylrest in 3/5-Stellung, einem Alkylmercaptorest in 3-Stellung bekannt sind (vgl. außer der obengenannten Patentpublikation GB-A1127 085, DE-A 1 644 177, DE-A 2 605 622, DE-A 2 137 719, DE-A 2 340 569, DD-A 142 542), sind die Verbindungen der Formel I bislang nicht in der Literatur beschrieben worden.

Man erhält die neuen Anilinderivate auf einfache Weise, wenn man Verbindungen der Formel

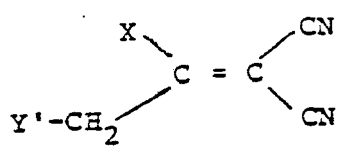

(II)

worin

Y′ für S-Alkyl oder —$CO_2R$ steht, mit Verbindungen der Formel

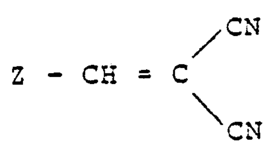

(III)

in an sich bekannter Weise kondensiert und aromatisiert sowie gegebenenfalls in dem Reaktionsprodukt den Rest S-Alkyl zum entsprechenden Alkylsulfonylrest oxidiert oder den Rest COOR zum Carbonsäurerest verseift und diesen durch Umsetzung mit Brom durch einen Bromsubstituenten ersetzt.

Die Ausgangsprodukte II und III sind bekannt bzw. nach an sich bekannten Methoden erhältlich (vgl. J. pr. Chem. *313*, 678 (1971), Bl. chem. Soc. Japan *2*, 306 (1927) und DE-OS 2 340 569).

Die Kondensation von II mit III erfolgt durch Basenkatalyse in Gegenwart eines organischen Lösungsmittels bei Temperaturen von − 10 bis 40 °C, vorzugsweise von − 5 bis 25 °C.

In den meisten Fällen gelingt bereits unter den vorstehend genannten Bedingungen für die Kondensation auch die Aromatisierung. Anderenfalls ist eine kurzzeitige Nacherhitzung, zweckmäßigerweise bei Siedetemperatur des Lösungsmittels erforderlich.

Die Oxidation der Verbindungen mit Y = S-Alkyl sowie die Verseifung von Verbindungen mit Y = COOR erfolgt nach üblichen Methoden. Die Bromierung der Carbonsäure wird beispielsweise in salzsaurer Lösung durchgeführt.

In besonders vorteilhafter Weise erhält man die neuen Anilinderivate, wenn man die Synthese als Eintopfreaktion gestaltet, indem man ein Gemisch aus

a) 1 Äquivalent einer Verbindung der Formel

$$X{-}CO{-}CH_2{-}Y' \quad (IV)$$

b) 1 Äquivalent einer Verbindung der Formel

$$Z{-}CH = O \quad (V)$$

und

c) 2 Äquivalenten Malodinitril in Gegenwart einer Base umsetzt.

Vorzugsweise wird 1 Äquivalent der Base (z. B. Morpholin) bei 0 °C zugesetzt und die Reaktion durch Rühren bei Raumtemperatur zu Ende geführt. In Ausnahmefällen ist eine Erwärmung auf Temperaturen von 60-100 °C vorzunehmen.

Obwohl die Herstellung der neuen Anilinderivate (I) zum großen Teil nach an sich bekannten Methoden erfolgt, ist der glatte Reaktionsverlauf als überraschend zu bezeichnen, da beispielsweise durch die Verwendung des Estersubstituenten $Y = CO_2R$ das Auftreten von Nebenreaktionen nicht auszuschließen war (vgl. Pyridonbildung gemäß Austr. J. Chem. *28*, 581 (1975)).

Es ist weiterhin bekannt, daß man an ortho-Cyananiline — ganz allgemein — Schwefelwasserstoff anlagern und durch anschließende Dehydrierung zu Aminobenzisothiazolen gelangen kann, die ihrerseits ebenfalls Diazokomponenten zum Aufbau interessanter Azofarbstoffe sind (vgl. DE-A 16 44 169). Unter zahlreichen anderen Diazokomponenten wird dort auch das 3-Amino-5-chlor-7-cyano-2,1-benzisothiazol erwähnt — jedoch ohne konkrete Angaben über dessen Eigenschaften und Herstellung.

Es wurde nun weiterhin gefunden, daß man die Aniline der Formel I durch $H_2S$-Anlagerung (zum Monothioamid) und anschließenden oxidativen Ringschluß in ähnliche Aminobenzisothiazole der Formel

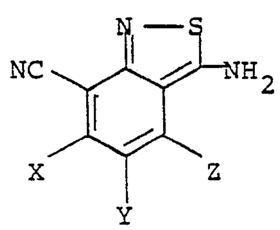

bzw.

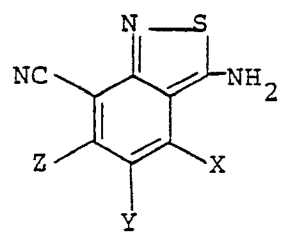

(VIa) (VIb)

überführen kann.

Die Herstellung der Aminobenzthiazole (VI) erfolgt dabei nach an sich bekannten Methoden (vgl. DE-A 1 544 375 = GB-AI 112 146, DE-A 2 412 975 = GB-AI 493 037, DE-A 1 644 169 und DE-A 2 617 807).

Je nach Substitutionsmuster der als Ausgangsmaterialien verwendeten Aniline (I) entstehen die Verbindungen VIa oder VIb oder Mischungen der beiden.

Anders ist die Situation bei dem Einsatz der bevorzugten Verbindungen der Formel I mit Z = H, wobei aller Wahrscheinlichkeit nach ausschließlich das VIa-Isomere entsteht.

Verbindungen der Formel VI mit $Y = SO_2$-Alkyl können im übrigen auch durch nachträgliche Oxidation von Verbindungen der Formel mit Y = S-Alkyl hergestellt werden, wobei die Oxidation zum Sulfon gegebenenfalls als Eintopfreaktion zusammen mit dem oxidativen Ringschluß durchgeführt werden kann.

Die Aminobenzisothiazole VI eignen sich ebenfalls zum Aufbau von Azofarbstoffen, insbesondere Dispersionsfarbstoffen, welche sehr farbstark sind und auf synthetischen Fasern violette bis blaue Färbungen mit hohem Echtheitsniveau ergeben.

Herstellungsbeispiele

Beispiel 1

Darstellung von

(Siehe Figur Seite 4 f.)

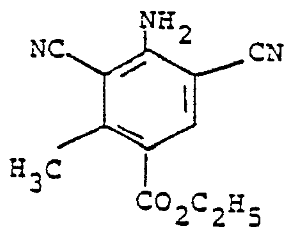

Zu 65 g Acetessigsäureethylester tropft man bei 0 °C in 30 Min. eine Lösung von 37,5 ml 37-proz. wäßrigem Form-aldehyd in 50 ml Methanol zu ; anschließend läßt man bei 0 °C eine Lösung von 66 g Malodinitril in 50 ml Methanol zutropfen. Man läßt 30 Min. nachrühren und läßt dann in 2,5 Stunden bei 0 °C eine Lösung von 43,3 ml Morpholin in 50 ml Methanol zulaufen. Man rührt 12 Stunden bei 0 °C und 48 Stunden bei Raumtemperatur. Man saugt ab und wäscht mit wenig Methanol. Ausbeute 50,5 g. Aus der Mutterlauge können nach 12-stündigem Kochen am Rückflußkühler und Animpfen weitere 4,6 g gewonnen werden.

Fp. : 149 °C ; $m_e$ + : 229 ;

1H-N.M.R (DMSO-$d_6$/TMS) : δ = 1,35 (t, 3H) ;

2,65 (s, 3H) ; 4,26 (q, 2H) ; 7,17 (s, 2H) ; 8,02 (s, 1H).

Beispiel 2

Darstellung von

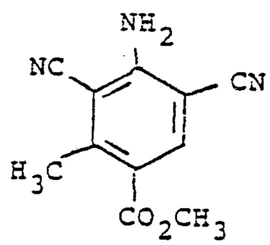

Zu 43,5 g Acetessigsäuremethylester tropft man bei 0 °C eine Lösung von 28,1 ml 37-proz. wäßrigem Formaldehyd in 25 ml Methanol und läßt anschließend eine Lösung von 33 g Malodinitril in 25 ml Methanol zulaufen. Bei 0 °C wird in 1,5 Stunden eine Lösung von 28 g Triethylendiamin (1,4-Diazabicyclo [2,2,2] octan) in 25 ml Methanol zugetropft. Man rührt weitere 20 Stunden bei 0 °C und erwärmt dann 7 Stunden zum Sieden am Rückflußkühler. Nach Abkühlen wird abgesaugt und mit 20 ml Methanol nachgewaschen. Ausbeute 16,2 g

$m_e$ + : 215 ; Fp : 146 °C.

Beispiel 3

Darstellung von

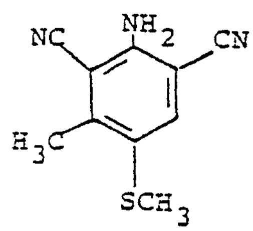

Zu 27,1 g 96-proz. 1-Methylmercapto-2-propanon läßt man nacheinander bei 0 °C eine Lösung von 18,8 ml 37-proz. wäßrigem Formaldehyd in 25 ml Methanol, eine Lösung von 33 g Malodinitril in 50 ml Methanol und eine Lösung von 21,65 ml Morpholin in 25 ml Methanol zutropfen. Man rührt 5 Stunden bei 0 °C, 48 Stunden bei Raumtemperatur und saugt dann 6,8 g schwach gelbliche Kristalle vom Fp. 164 °C ab. $m_e$ + : 203. Das Filtrat wird 5,5 Stunden zum Sieden erhitzt. Nach Abkühlen saugt man weitere 6,7 g 2,6-Dicyan-3-methyl-4-methylmarcaptoanilin ab.

Beispiel 4

Analog dem Verfahren von Beispiel 2 erhält man durch Reaktion von Acetessigsäureethylester mit Acetaldehyd und Malonitril 2,6-Dimethyl-3,5-dicyan-4-amino-benzoesäureethylester. $m_e$ + : 243 Fp. 206 °C.

Beispiel 5

Darstellung von

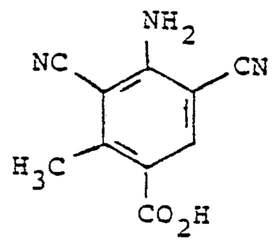

Eine Suspension von 48,8 g 2-Methyl-3,5-dicyan-4-aminobenzoesäureethylester in 300 ml konz. Salzsäure wird 20 Stunden am Rückflußkühler gekocht. Man saugt ab und wäscht mit Wasser neutral. Ausbeute 36 g. Durch Lösen in verd. Natronlauge und Ausfällen mit verd. Salzsäure kann das Produkt nachgereinigt werden.

IR ; 2 220 $cm^{-1}$ (CN) ; Fp. 200 °C ;
$^1$H-N.M.R. (DMSO-$d_6$/TMS) ; $\delta$ = 2,68 (s, 3H) ;
7,15 (s, 3H) ; 8,13 (s, 1H).

Beispiel 6

Darstellung von

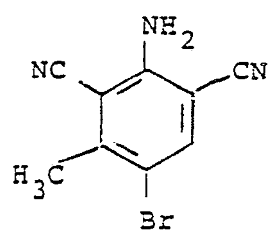

Zu einer Suspension von 10,05 g 2-Methyl-3,5-dicyan-4-amino-benzoesäure in 250 ml konz. Salzsäure tropft man bei Raumtemperatur unter Rühren 8 g Brom zu und setzt nach 48 Stunden nochmals 8 g Brom zu. Man rührt nochmals 48 Stunden und erwärmt anschließend 3 Stunden auf 50 °C. Nach Abkühlen saugt man 11,8 g 2,6-Dicyan-3-methyl-4-brom-anilin ab.

$m_e$ + : 235 (100 %), 237 (97 %) ;
Fp. 212 °C.

Beispiel 7

Herstellung des 3-Amino-5-carboethoxy-6-methyl-7-cyan-2,1-benzisothiazols

A. Herstellung des Thioamids der Formel

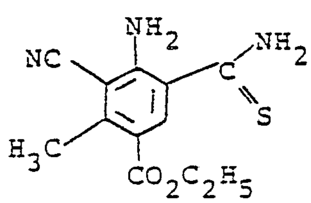

Eine Suspension von 18,3 g 2-Methyl-4-amino-3,5-dicyanbenzoesäureethylester in 100 ml Methanol wurde mit 100 ml 40 proz. Ammoniumsulfidlösung versetzt und 18 Stunden bei 25 °C gerührt. Es wurde abgesaugt und mit Wasser gewaschen.

Ausbeute : 18,5 g Fp. 202 °C.
$M_e$ $^+$ : 263 (67,5 %)

B. Herstellung des Benzisothiazols der Formel

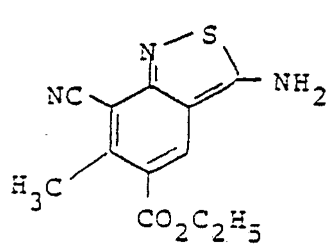

Eine Suspension von 18,4 g 2-Methyl-3-cyan-4-amino-5-thiocarbonamido-benzoesäureethylester (oder isomerer 2-Methyl-3-thiocarbonamido-4-amino-5-cyan-benzoesäureethylester) in 75 ml Eisessig werden mit 10,85 g 35 proz. Wasserstoffperoxid unter Rühren versetzt. Die Temperatur steigt bis auf 60 °C und die Farbe der Suspension schlägt nach Gelb um. Man läßt 30 Minuten nachrühren, saugt ab und wäscht mit Eisessig und Wasser. Ausbeute : 17,9 g (98 % d. Th.) $M_e$ + : 261 (100 %).

Verwendungsbeispiel

Zu einer Suspension von 7,57 g 3-Amino-5-carboethoxy-6-methyl-7-cyan-2,1-benzisothiazols in 40 ml Propionsäure und 80 ml Eisessig tropfte man bei 0 °C 4,8 ml 42 proz. Nitrosylschwefelsäure. Es wurde eine weitere Stunde bei 0 °C gerührt und dann geklärt. Die Lösung der Diazotierung ließ man bei 0 °C in eine Lösung von 7,14 g 3-Diethyl-aminoacetanilid in 100 ml Eisessig und 20 ml 10 proz. Aminosulfonsäurelösung einlaufen. Nach Rühren über Nacht wurde abgesaugt, mit Wasser gewaschen und getrocknet. Man erhält 12 g eines Farbstoffs, der Polyester in einem klaren Blauton färbt.

**Patentansprüche**

1. Anilinderivate der Formel

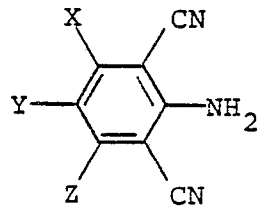

worin

X Alkyl,

Y —S-Alkyl, —$SO_2$-Alkyl, —$CO_2$—R oder — falls Z = H — auch Brom und

Z Wasserstoff oder Alkyl bedeuten,

wobei

R für Alkyl, Cycloalkyl oder Wasserstoff steht und die Alkylreste 1-6, vorzugsweise 1-4 C-Atome und die Cycloalkylreste 5 oder 6 Ring-C-Atome aufweisen.

2. Aniliderivate gemäß Anspruch 1, dadurch gekennzeichnet, daß

X Methyl und

Z Wasserstoff bedeuten.

3. Anilinderivate gemäß Anspruch 1 oder 2, dadurch gekennzeichnet, daß

Y —$CO_2$-$C_1$-$C_6$-Alkyl bedeutet.

4. Anilinderivat gemäß Anspruch 1 der Formel

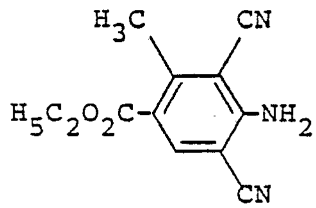

5. Aminobenzisothiazole der Formel

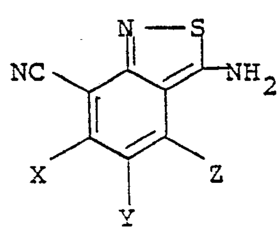

bzw.

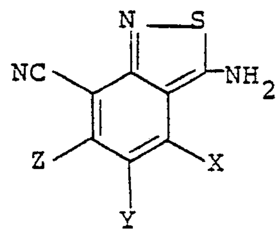

worin X, Y und Z die in Anspruch 1 genannten Bedeutungen haben.

6. Aminobenzisothiazole der Formel

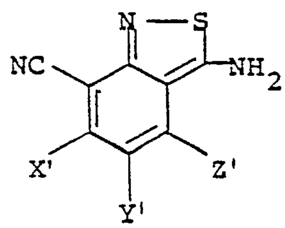

bzw.

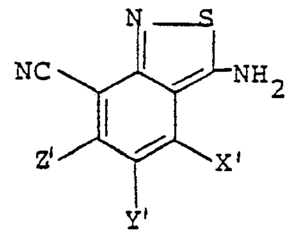

worin X' Methyl, Y' —$CO_2$-$C_1$-$C_6$-Alkyl und Z' Wasserstoff bedeuten.

**Claims**

1. Aniline derivatives of the formula

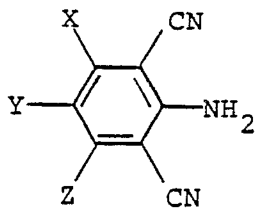

wherein
X denotes alkyl,
Y denotes —S-alkyl, —$SO_2$-alkyl, —$CO_2$—R, or — if Z = H — bromine, and
Z denotes hydrogen or alkyl,
where
R represents alkyl, cycloalkyl, or hydrogen, and the alkyl radicals have 1-6, preferably 1-4, C atoms, and the cycloalkyl radicals have 5 or 6 ring C atoms.

2. Aniline derivatives according to Claim 1, characterised in that
X denotes methyl, and
Z denotes hydrogen.

3. Aniline derivatives according to Claim 1 or 2, characterised in that
Y denotes —$CO_2$-$C_1$-$C_6$-alkyl.

4. Aniline derivative according to Claim 1, of the formula

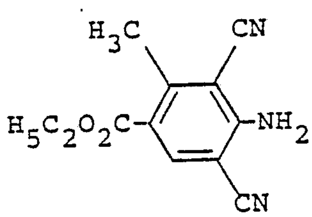

5. Aminobenzisothiazoles of the formula

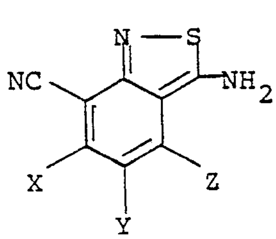

or

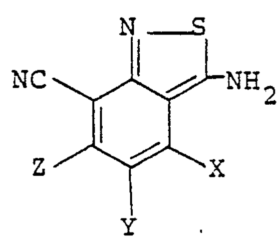

wherein
X, Y and Z have the meanings mentioned in Claim 1.

6. Aminobenzisothiazoles of the formula

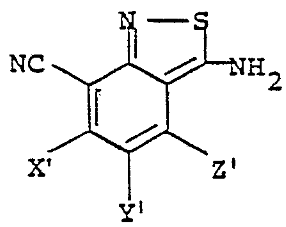

or

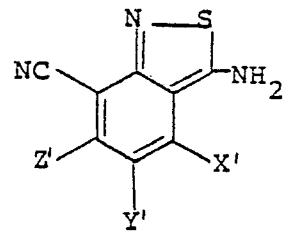

wherein

X'denotes methyl,

Y' denotes $-CO_2$-$C_1$-$C_6$-alkyl and

Z' denotes hydrogen.

**Revendications**

1. Dérivés d'aniline de formule

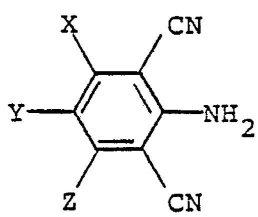

dans laquelle

X est un groupe alkyle,

Y est un groupe —S-alkyle, $-SO_2$-alkyle, $-CO_2-R$ ou — au cas où Z représente H — également le brome et

Z est l'hydrogène ou un groupe alkyle,

R représentant un groupe alkyle, cycloalkyle ou l'hydrogène et les restes alkyle présentant 1 à 6, de préférence 1 à 4 atomes de carbone et les restes cyclo-alkyle 5 ou 6 atomes de carbone dans le noyau.

2. Dérivés d'aniline suivant la revendication 1, caractérisés en ce que

X est un groupe méthyle et

Z est l'hydrogène.

3. Dérivés d'aniline suivant la revendication 1 ou 2, caractérisés en ce que

Y désigne un groupe $-CO_2$-(alkyle en $C_1$ à $C_6$).

4. Dérivé d'aniline suivant la revendication 1, de formule

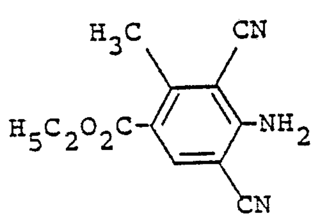

5. Aminobenzisothiazoles de formule

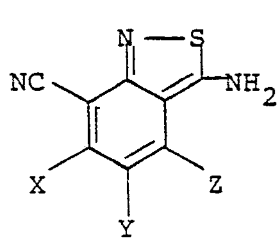

ou

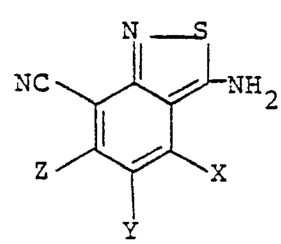

où X, Y et Z ont les définitions mentionnées dans la revendication 1.

6. Aminobenzisothiazoles de formule

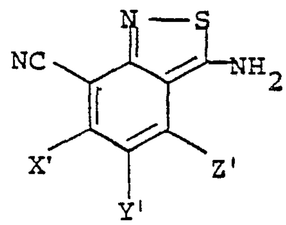

ou

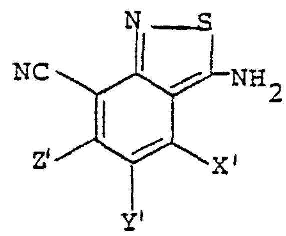

où X' est un groupe méthyle, Y' est un groupe —$CO_2$-(alkyle en $C_1$ à $C_6$) et Z' représente l'hydrogène.